# FASCICULE DE BREVET EUROPEEN

(11) **EP 2 753 268 B1**
(45) Date de publication et mention de la délivrance du brevet: **27.04.2016**
(21) Numéro de dépôt: 12767037.0
(22) Date de dépôt: 06.09.2012
(51) Int. Cl.: A61F 2/20

(54) **NOUVELLE PROTHESE INTRALARYNGEE**
NEUE INTRALARYNGEALE ENDOPROTHESE
NEW INTRA-LARYNGEAL ENDOPROSTHESIS

(30) Priorité: 06.09.2011 FR 1102694
(43) Date de publication de la demande: 16.07.2014
(73) Titulaire: Protip, 67000 Strasbourg (FR)
(72) Inventeur: WALDER, André Michel Charles, F-94240 L'Hay-les-Roses (FR); LEFEBVRE, Sylvain, F-25480 Vuillafans (FR); PERRIN, Nicolas, F-67300 Schiltigheim (FR); BERENGER, Maurice, F-67000 Strasbourg (FR)
(74) Mandataire: Flesselles, Bruno F.G.
(86) Numéro de dépôt international: PCT/FR2012/051996
(87) Numéro de publication internationale: WO 2013/034858

(56) Documents cités:
- EP-A1- 0 815 807
- WO-A2-02/083031
- FR-A1- 2 891 133
- FR-A1- 2 924 331

## Description

La présente invention se rapporte au domaine des prothèses avec pour fonction la restauration à la fois de la déglutition, de la respiration et de la phonation chez un patient ayant un larynx dysfonctionnel. Plus particulièrement, la présente invention concerne un dispositif à clapets formant prothèse intralaryngée destinée à être implantée au sein d'un larynx anatomique en place dont la fonction consiste à permettre la respiration tout en assurant l'étanchéité vis-à-vis d'éléments comme la salive, le mucus ou tout autre élément issu du bol alimentaire. La prothèse intralaryngée complète ainsi que ses différentes applications, font également l'objet de la présente invention.

Il existe plusieurs pathologies ou conséquences de pathologies et/ou de traitements qui conduisent les patients à présenter des dysfonctionnements du larynx. Certains de ces événements conduisent à une paralysie partielle ou totale, qui est soit provisoire soit définitive. Le larynx est un conduit ostéo-cartilagineux dont le rôle principal est d'ordre respiratoire. Tout dysfonctionnement peut donc compromettre la fonction respiratoire chez le patient. Le larynx a un rôle important dans le processus de déglutition dans le sens où sa fermeture protège les voies aériennes inférieures. Tout dysfonctionnement entraîne donc des problèmes de pénétration d'éléments indésirables, liquides ou solides, dans les voies aériennes inférieures.

Pour remédier au dysfonctionnement du larynx, l'art antérieur ne propose que peu de solutions car les fonctions du larynx sont multiples et que ceci nécessite des prothèses complexes dont le positionnement implique un acte chirurgical. Il faut donc que le dispositif soit adapté aux différentes fonctions du larynx mais qu'il présente également une durée de vie substantielle en conditions physiologiques afin de limiter les interventions du chirurgien. Par ailleurs, le dispositif doit être réalisé dans un matériau biocompatible, résistant, léger, durable, et qu'il présente la forme la plus adaptée au lieu d'implantation afin de causer le moins de gêne au patient.

La demande de brevet EP 0815807 décrit une prothèse destinée à remédier au dysfonctionnement du larynx, ladite prothèse consiste en un tube en silicone présentant au niveau de l'une de ses extrémités conformée en biseau, d'une part une face d'obturation inclinée et, d'autre part, des orifices latéraux de communication entre l'intérieur et l'extérieur du tube formant la prothèse. En pratique, la face d'obturation inclinée dirige le bol alimentaire de la bouche vers l'oesophage alors que les orifices latéraux de communication permettent la circulation d'air dans les deux sens. Il n'en reste pas moins vrai que par ceux-ci peuvent se produire des fuites et, plus particulièrement, des introductions de fluides ou de reflux de fluides ou de tout autre élément du bol alimentaire vers les voies aériennes inférieures. En effet, dans la pratique, l'orientation de la face d'obturation n'est pas suffisante pour assurer à elle seule une totale étanchéité.

La demande de brevet EP 08872036, publié comme EP2240120, décrit également un système d'implant destiné à restaurer les fonctions respiratoire, phonatoire et de déglutition pour un patient dont le larynx est dysfonctionnel. Cette demande décrit un dispositif à clapets réalisé au moins en partie dans un matériau semi-rigide, biocompatible, léger et résistant, par exemple du silicone. Le dispositif décrit est préférentiellement constitué d'une armature en silicone, de clapets en silicone et d'une bague périphérique de renfort en titane qui est noyée dans le silicone de l'armature. L'originalité de ce dispositif réside dans un système à double clapets dont l'objectif est de permettre la respiration ainsi que la déglutition en formant un obturateur parfaitement étanche grâce à une découpe en biseau des circonférences des deux clapets de forme circulaire ainsi que de la circonférence de la surface interne de l'armature porteuse annulaire. Ce dispositif présente une durée de vie limitée due à la déformation des clapets en conditions physiologiques ainsi qu'à l'apparition de moisissures liées à la présence du silicone qui est facilement colonisé par des cellules non désirées. Le remplacement du dispositif à plus ou moins court terme est donc nécessaire.

Ces dispositifs de l'art antérieur ont pour défaut majeur d'être sensibles à l'usure et à la colonisation en conditions physiologiques, notamment par des moisissures. En effet, toute déformation de l'obturateur entraîne inévitablement un défaut d'étanchéité et donc un mauvais fonctionnement du dispositif visant à pallier le dysfonctionnement du larynx. Il est alors nécessaire qu'un chirurgien intervienne pour remplacer le ou les éléments défectueux dans les dispositifs de l'art antérieur.

La présente invention vise à remédier à ces inconvénients en proposant un nouveau dispositif permettant, d'une part d'assurer une parfaite étanchéité du dispositif en empêchant toute fuite vers les voies aériennes inférieures et, d'autre part, de permettre une circulation de l'air dans les deux sens afin de permettre la respiration du patient ainsi que les fonctions de phonation et de déglutition. De plus, l'originalité de cette invention qui est détaillée ci-après, réside dans le fait que le dispositif selon l'invention présente une excellente résistance à l'usure et à la déformation en conditions physiologiques et présente une durée de vie plus importante que les dispositifs de l'art antérieur.

Pour ce faire, la présente invention propose une nouvelle approche en rapport avec le dispositif à clapets décrit dans la demande de brevet EP 08872036 (EP2240120). Afin de développer un dispositif plus résistant et performant en milieu physiologique, les inventeurs ont procédé à l'usinage de pièces en métal biocompatible, qui sont combinées entre elles pour former un obturateur étanche. Les métaux biocompatibles connus à ce jour sont le titane ou les alliages à base de titane, l'or ou les alliages à base d'or ainsi que le platine ou les alliages à base de platine, le niobium et le tantale ou leurs alliages. Le dispositif réalisé en un matériau semi-rigide décrit dans la demande de brevet EP 08872036 est aisé à fabriquer par moulage ou injection/moulage par exemple, car les matériaux semi-rigides sont faciles d'usage et flexibles. Le fait de vouloir réaliser un dispositif à clapets en métal massif destiné à être implanté au sein d'un larynx dysfonctionnel est un réel défi technique. En effet, l'état de l'art dans le domaine des implants trachéaux, que ce soit par voie de trachéotomie ou par voie endo-buccale, tend plutôt vers l'utilisation de matériaux semi-rigides car ils peuvent être pliés à des fins d'insertion pour être le moins traumatiques possibles aussi bien au niveau des tissus d'implantation que des zones anatomiques qui vont être traversées avant mise en place du dispositif. Par contre dans le domaine des prothèses ou implants qui nécessitent de grandes contraintes mécaniques comme les implants osseux, on tend à utiliser des matériaux rigides y compris des matériaux métalliques. Cependant, lesdits implants ou prothèses ne sont pas en mouvement et sont là pour stabiliser ou favoriser le développement osseux. La présente invention va à l'encontre de l'état de l'art dans le domaine de la laryngologie en développant un dispositif à clapets, donc en mouvement, principalement réalisé en métal massif.

L'objet de l'invention est particulièrement innovant car il a une exceptionnelle durée de vie, il résiste particulièrement bien à la déformation et permet en outre, d'assurer la circulation de l'air lors des phases d'inspiration et d'expiration tout en protégeant les voies aériennes inférieures par obturation quand il y a lieu ; ainsi les fonctions de déglutition, de respiration et de phonation sont restaurées de façon durable.

Plus particulièrement, la présente invention concerne, de manière générale, un dispositif à clapets formant prothèse intralaryngée réalisé en métal massif biocompatible et destiné à être implanté au sein d'un larynx dysfonctionnel, ledit dispositif comportant une portion distale formant armature porteuse annulaire et une portion centrale formant obturateur destinée à permettre le passage de l'air et à empêcher de manière hermétique le passage de tout autre élément, ledit dispositif étant caractérisé en ce que l'obturateur comprend i) une partie périphérique formant un premier clapet solidaire de l'armature porteuse annulaire au niveau d'une première région charnière, et ii) une partie centrale formant un second clapet solidaire du premier clapet au niveau d'une seconde région charnière, lesdits premier et second clapets coopérant entre eux de manière totalement hermétique.

L'invention sera mieux comprise à la lumière des exemples ci-après ainsi que des figures suivantes, dans lesquelles les références entre parenthèses se rapportent aux figures jointes à la présente description.

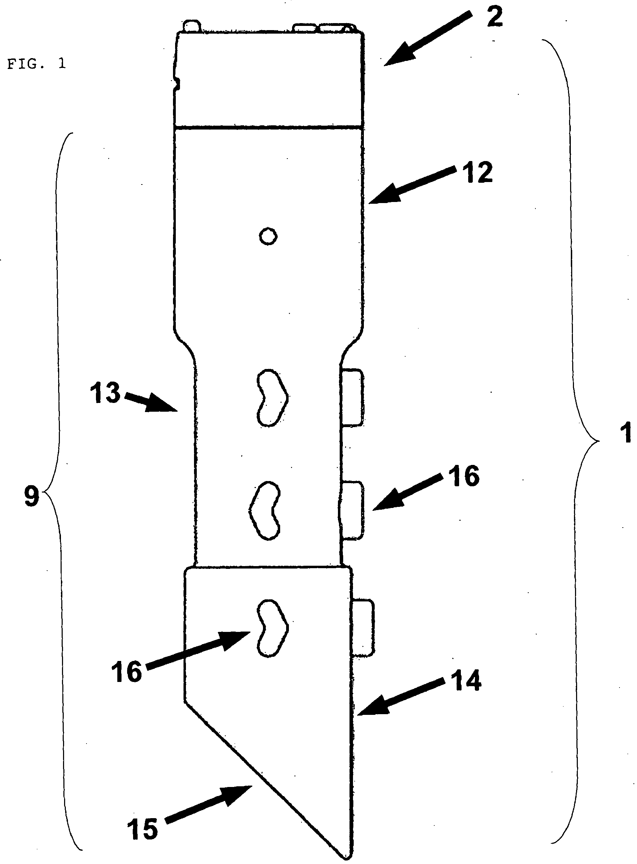

La Fig. 1 décrit une vue de profil d'un dispositif (1) formant prothèse intralaryngée pour larynx dysfonctionnel selon l'invention. On y distingue l'armature porteuse annulaire (2) dont la partie supérieure est équipée de clapets formant obturateur et la partie inférieure qui est équipée d'une jupe (9) en silicone, destinée à être mise en place dans le larynx, au sommet de la trachée. Sont également représentées les différentes parties formant la jupe (9), à savoir la première partie (12) fixée à l'armature porteuse annulaire, la seconde partie (13) et la troisième partie (14) qui est en forme de biseau (15). Sur la partie extérieure de la jupe (9) on voit des ergots (16) qui visent à bloquer la prothèse une fois implantée.

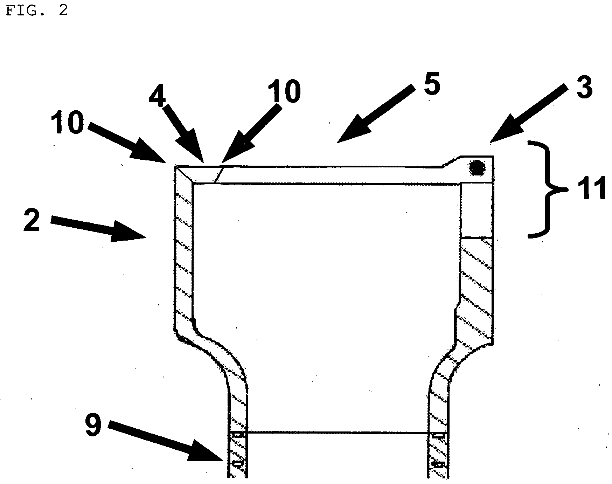

La Fig. 2 est une vue en coupe verticale de la partie supérieure d'un dispositif (1) formant prothèse intralaryngée pour larynx dysfonctionnel selon l'invention. On y distingue l'armature porteuse annulaire (2) dans la partie supérieure du dispositif (1) et la collerette ou jupe (9) fixée sur la partie inférieure de l'armature porteuse annulaire (2). Le grand clapet (4) et le petit clapet (5) sont portés par l'armature porteuse annulaire (2) et s'articulent autour d'une zone charnière (3). Dans ce mode de réalisation la zone charnière (3) est un axe rigide qui vient s'imbriquer dans une découpe réalisée dans les clapets (4) et (5). Des éléments de butée (10) permettent de bloquer les clapets selon le mode de réalisation choisi. Les clapets (4) et (5) en position fermée forment un obturateur (11).

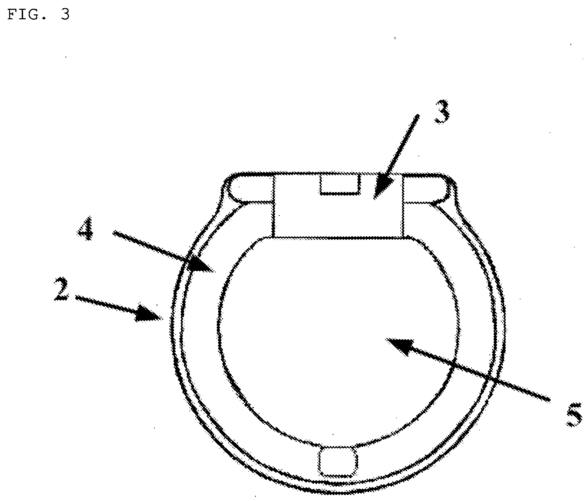

La Fig. 3 est une vue du dessus d'un dispositif (1) formant prothèse intralaryngée selon l'invention. On y distingue le grand clapet (4) et le petit clapet (5) ainsi que la circonférence de l'armature porteuse annulaire (2). On visualise également la zone charnière (3) qui correspond à un modèle tel que celui décrit dans la Fig. 2, à savoir une charnière à axe rigide.

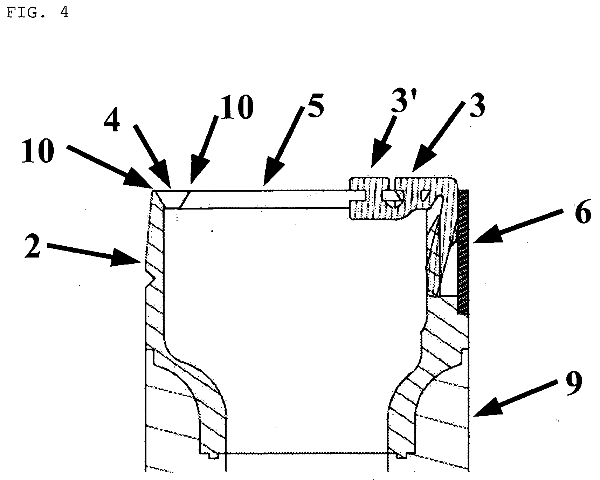

La Fig. 4 décrit une vue en coupe verticale de la partie supérieure du dispositif (1) formant prothèse intralaryngée pour larynx dysfonctionnel selon l'invention. Sont illustrés l'obturateur formé par les clapets (4, 5) en position horizontale, les butées (10), une première zone charnière (3), une seconde zone charnière (3'), une plaque de fixation (6) ainsi que l'armature porteuse annulaire (2) et la collerette ou jupe (9). Ce modèle détaille une zone charnière réalisée dans un matériau semi-rigide qui a la forme d'une languette fixée sur les clapets (4) et (5) à l'une de ses extrémités et bloquée par la plaque (6) à son autre extrémité. Les clapets (4) et (5) en position fermée forment un obturateur (11).

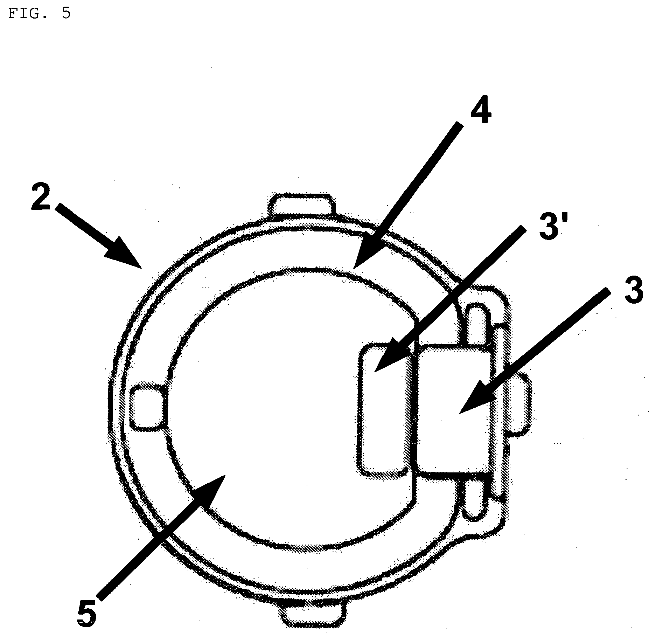

La Fig. 5 est une vue du dessus d'un dispositif (1) formant prothèse intralaryngée selon l'invention. On y distingue le grand clapet (4) et le petit clapet (5) ainsi que la circonférence de l'armature porteuse annulaire (2). On visualise également une première zone charnière (3) et une seconde zone charnière (3') qui correspond à un modèle tel que celui décrit dans la Fig. 4, à savoir une charnière semi-rigide.

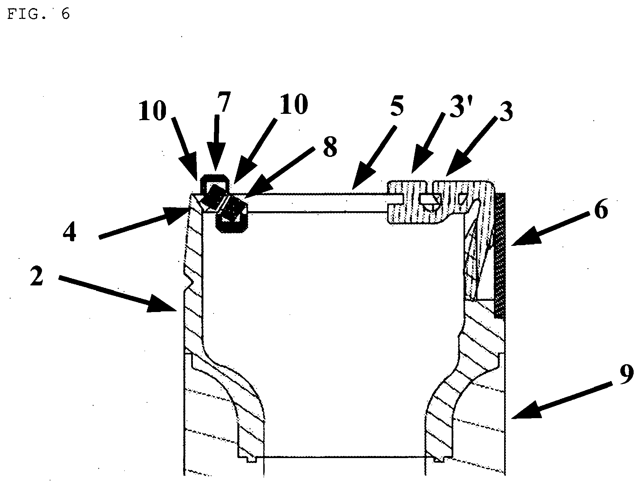

La Fig. 6 décrit une vue en coupe verticale de la partie supérieure du dispositif (1) formant prothèse intralaryngée pour larynx dysfonctionnel selon l'invention. C'est le modèle décrit dans les Fig. 4 et 5, à savoir un modèle à charnière semi-rigide. Sont illustrés l'obturateur formé par les clapets (4) et (5) en position horizontale, les butées (10), les zones charnières (3) et (3'), les éléments d'assistance (7, 8), la plaque de fixation (6) ainsi que l'armature porteuse annulaire (2) et la collerette ou jupe (9). Les éléments d'assistance favorisent la fermeture des clapets.

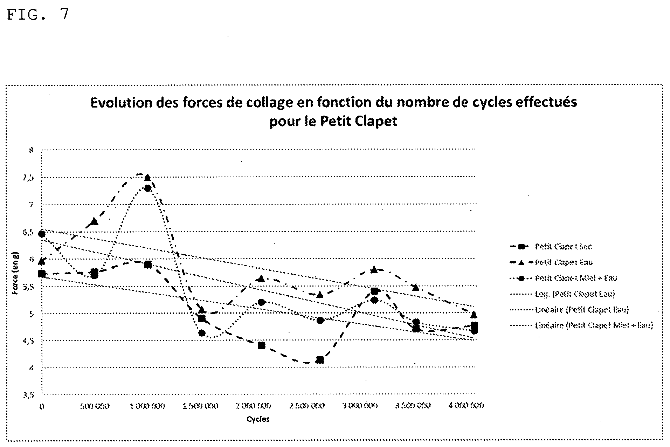

La Fig. 7 représente un graphique qui décrit l'évolution des forces de collage en fonction du nombre de cycles respiratoires effectués pour le petit clapet (5).

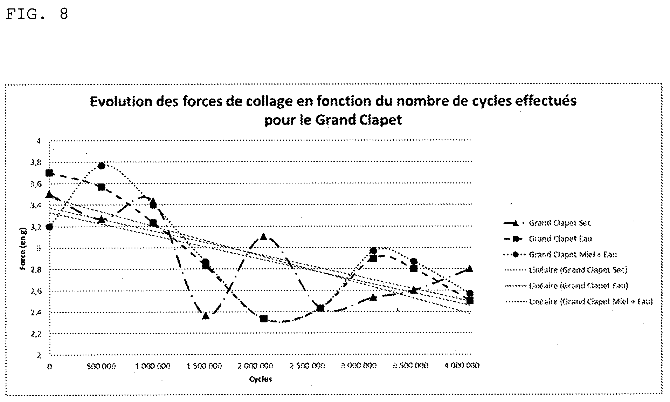

La Fig. 8 représente un graphique qui décrit l'évolution des forces de collage en fonction du nombre de cycles respiratoires effectués pour le grand clapet (4).

Le dispositif selon l'invention est constitué majoritairement de métal massif biocompatible tel le titane ou les alliages à base de titane, l'or ou les alliages à base d'or ainsi que le platine ou les alliages à base de platine, le niobium et le tantale ou leurs alliages ou de manière plus générale tout autre métal massif biocompatible. Dans un mode de réalisation préféré le métal utilisé pour la fabrication de l'armature porteuse annulaire (2) ainsi que les clapets (4) et (5) est du titane ou un alliage à base de titane. En effet ce matériau est parfaitement biocompatible et la fabrication de pièces de petite taille dont l'épaisseur est facilement maîtrisable est possible. La présente invention décrit un dispositif à clapets (1) destiné à être implanté au sein d'un larynx dysfonctionnel, au moyen d'une jupe en silicone (9) ledit dispositif comportant une portion distale formant armature porteuse annulaire (2) et une portion centrale formant obturateur (11) destiné à permettre le passage de l'air et à empêcher de manière hermétique le passage de tout autre élément, l'obturateur (11) comprend i) une partie périphérique formant un premier clapet (4) solidaire de l'armature porteuse annulaire (2) au niveau d'une première région charnière (3), et ii) une partie centrale formant un second clapet (5) solidaire du premier clapet au niveau d'une seconde région charnière (3'), ainsi qu'un moyen formant collerette ou jupe (9) pour la fixation et le retrait de la prothèse intralaryngée au sein d'un larynx dysfonctionnel situé en dessous de l'armature porteuse annulaire (2), dans lequel l'armature porteuse annulaire (2) ainsi que les deux clapets (4) et (5) formant obturateur (11) sont en métal massif, préférentiellement en titane ou en alliage à base de titane.

La méthode de fabrication du dispositif selon la présente invention nécessite des compétences microtechniques. En effet, afin d'assurer une parfaite étanchéité du dispositif selon l'invention et plus particulièrement de l'obturateur, les différents éléments en métal massif doivent être parfaitement ajustés et le fonctionnement des clapets doit être extrêmement précis et fiable dans la répétition des mouvements. Pour ce faire, chaque pièce est réalisée avec un soin particulier avant d'être assemblée aux autres éléments formant le dispositif selon l'invention. La zone charnière revêt une importance toute particulière dans la mesure où son rôle est de permettre l'abaissement et le soulèvement des clapets afin de restaurer les fonctions du larynx dysfonctionnel dans lequel le dispositif est inséré. Ce rôle est répétitif et d'une grande précision. En effet, sans fonction charnière fiable, le dispositif resterait bloqué soit en position ouverte (pas de protection des voies aériennes inférieures), soit en position fermée (sans possibilité de circulation de l'air), soit les clapets seraient désaxés entrainant l'absence d'étanchéité à leur fermeture, entraînant des complications sérieuses pour la patient. Dans la présente invention, la zone charnière doit permettre l'ouverture de clapets en métal massif et le rappel de ceux-ci en position fermée grâce à une force de rappel qu'elle exerce. La zone charnière est donc une zone d'articulation qui est soit liée aux propriétés mécaniques du matériau utilisé, soit liée au fonctionnement d'un mécanisme permettant le mouvement.

Dans la présente description, les expressions « clapet », « valve », « obturateur » ou encore « soupape » peuvent être utilisées de manière interchangeable pour désigner un élément mobile capable de passer d'une position fermée, ou d'obturation, à une position ouverte permettant le passage de l'air.

Les expressions « distales » et « centrales » ont pour référentiel le centre du dispositif selon l'invention. Il s'en suit que l'expression « portion distale » fait référence à la portion la plus éloignée du centre du dispositif par opposition à l'expression « portion centrale » qui fait référence à la portion la plus proche du centre dudit dispositif selon l'invention.

Les termes « semi-rigide » et « super élastique » sont employés en référence à des matériaux souples présentant des propriétés spontanées et permanentes de rigidité ou des matériaux rigides présentant des propriétés spontanées et permanentes d'élasticité. De manière générale, ces matériaux permettent une certaine élasticité sans déformation permanente, en conséquence ils reprennent spontanément leur forme initiale. On citera à titre d'exemple de matériaux semi-rigides les plastiques, la gomme, le silicone et comme exemple de matériau super élastique le nitinol (alliage nickel-titane).

Selon une forme de réalisation préférée, la structure circulaire des deux clapets est préférable du fait que, pour un encombrement donné c'est cette conformation qui offre la plus grande ouverture pour le passage de l'air. Cependant, une telle forme n'est aucunement limitative et le dispositif selon l'invention peut présenter d'autres formes ou structures notamment de section ovoïde.

Au repos, les clapets du dispositif selon l'invention sont imbriqués l'un dans l'autre et dans le même plan. Cependant, suivant le mode de coopération choisi, l'ensemble des deux clapets s'abaisse lors de l'inspiration alors que seul le plus petit clapet se soulève lors de l'expiration, ou alors l'ensemble des deux clapets se soulève lors de l'expiration alors que seul le plus petit clapet s'abaisse lors de l'inspiration.

Parmi les modes de réalisation possibles, les deux clapets peuvent voir leur course bloquée par une butée (10) ou par un système de type magnétique. Il est également envisageable de combiner des éléments de butée (10) et un système de type magnétique.

Une autre formulation consiste à dire que l'invention repose sur la coopération de deux clapets (4) et (5) imbriqués l'un dans l'autre dans le même plan au repos, l'ensemble des deux clapets s'abaissant à l'inspiration alors que seul le plus petit s'ouvre à l'expiration ou alors l'ensemble des deux clapets s'ouvrant à l'expiration alors que seul le clapet central s'abaisse à l'inspiration. Lorsque les deux clapets (4) et (5) sont dans le même plan au repos, ils forment un obturateur (11) qui est hermétique.

Le fonctionnement des clapets (4) et (5) est réalisé par la surpression et la dépression dans les poumons pendant les phases d'expiration et d'inspiration. Dans l'un des modes de réalisation, lors de l'inspiration et du fait de la dépression existant dans les poumons, les deux clapets (4) et (5) s'abaissent dans le même mouvement. A l'expiration, du fait de la surpression existant dans les poumons seul le petit clapet (5) s'ouvre vers l'extérieur du dispositif, le grand clapet (4) restant bloqué sur son siège. Dans un autre mode de réalisation de la présente invention, lors de l'inspiration et du fait de la dépression existant dans les poumons, le petit clapet central (5) s'abaisse seul, le grand clapet (4) restant bloqué sur son support. A l'expiration, du fait de la surpression existant dans les poumons, les deux clapets (4) et (5) se soulèvent vers l'extérieur du dispositif dans le même mouvement. La garantie d'une bonne étanchéité de la trachée à la fermeture des clapets (4) et (5) combinée à une grande résistance mécanique dans le dispositif selon l'invention, est un élément très important pendant la déglutition pour éviter aux aliments ou à la salive de pénétrer dans la trachée puis dans les poumons.

Dans un mode de réalisation particulier, le clapet (4) est capable de s'abaisser sous l'effet de la dépression résultant de l'inspiration du patient et le clapet (5) est capable i) de coopérer avec le clapet (4) en s'abaissant mais également ii) de se soulever sous l'effet de la surpression exercée par l'air expiré par le patient. Dans un mode de réalisation particulier du dispositif (1) selon l'invention, la partie interne du clapet (4) comprend au moins un élément de butée (10) empêchant le soulèvement du clapet (5).

Dans un autre mode de réalisation particulier, le clapet (4) est capable de se soulever sous l'effet de la surpression exercée par l'air expiré par le patient et que le clapet (5) est capable i) de coopérer avec le clapet (4) en se soulevant mais également ii) de s'abaisser sous l'effet de la dépression résultant de l'inspiration du patient. Dans un mode de réalisation particulier du dispositif (1) selon l'invention, la partie interne de l'armature porteuse annulaire (2) comprend au moins un élément de butée (10) empêchant l'abaissement du clapet (4).

Dans un mode de réalisation préféré du dispositif (1) selon l'invention, l'élément de butée (10) consiste en ce que la circonférence externe du premier clapet (4) de l'obturateur (11) soit découpée en forme de biseau « vers le bas » et en ce que la circonférence interne de l'armature porteuse annulaire (2) se trouvant en vis-à-vis soit également découpée en biseau « inversé vers le haut », les deux découpes en biseau coopérant entre elles de manière à bloquer le soulèvement du premier clapet (4).

Comme il ressortira des exemples ci-dessous, une des caractéristiques de l'invention repose sur le matériau utilisé pour la fabrication de l'armature porteuse annulaire (2) et des clapets (4) et (5) du dispositif (1) selon l'invention. Cet ensemble est également appelé partie active du dispositif. L'épaisseur des clapets (4) et (5), leur système d'articulation et leur forme générale permettant une étanchéité parfaite sont également des éléments déterminants pour un fonctionnement optimal du dispositif (1). En effet, le matériau préféré étant le titane massif, les clapets (4) et (5) ne présentent pas d'élasticité contrairement à ceux décrits dans l'art antérieur. Le rôle des régions charnière (3) et (3') est donc primordial afin d'assurer un mouvement régulier et suffisant en fonction des sollicitations du patient. En effet la zone charnière (3), quelle que soit sa configuration selon la présente invention, influe directement sur la capacité du dispositif (1) à s'abaisser ou bien à maintenir sa position. Cette élasticité doit être évaluée de manière à pouvoir résister à la pression correspondant à la poussée exercée par les aliments ou par une accumulation de salive, de mucus ou de tout autre fluide, pour éviter leur pénétration dans les voies aériennes supérieures, tout en étant capable de s'abaisser ou de se soulever suite à une surpression expiratoire ou à une dépression inspiratoire.

Dans un mode de réalisation préféré, les régions charnières (3) et (3') sont situées entre les clapets (4) et (5) et l'armature porteuse annulaire (2) pour ne former qu'une seule zone charnière (3, 3') pour les deux clapets (4) et (5). Enfin dans un mode de réalisation particulier, les régions charnières (3) et (3') sont constituées d'un matériau semi-rigide, super élastique ou rigide. Dans le cas d'une zone charnière réalisée dans un matériau rigide, celui-ci est par exemple un axe métallique autour duquel vient s'articuler le clapet correspondant grâce à des moyens prévus à cet effet lors de la découpe de la pièce (voir Fig. 2). Dans le cas d'une zone charnière réalisée dans un matériau semi-rigide ou super élastique, celui-ci est par exemple du silicone (voir Fig. 4) ou du nitinol.

Dans la suite de la description, l'expression « bol alimentaire » sera utilisée pour définir non seulement tout élément dudit bol alimentaire, mais également le mucus, la salive ou tout autre élément ou corps étranger aux voies respiratoires supérieures et inférieures.

Selon un mode de réalisation préféré de l'invention, le dispositif est caractérisé en ce que l'armature porteuse annulaire (2) comprend au moins un élément de butée (10) empêchant l'abaissement du premier clapet (4). Cet élément de butée (10) joue un rôle clé car il permet soit d'empêcher tout mouvement du premier clapet (4) vers le bas suite à l'inspiration du patient, soit d'empêcher tout mouvement du premier clapet (4) vers le haut suite à l'expiration du patient.

Ledit élément de butée (10) peut consister en tout moyen venant entraver et bloquer le mouvement du premier clapet (4) vers le haut ou vers le bas, comme par exemple un ergot faisant saillie sur l'ensemble de la circonférence interne de l'armature porteuse annulaire ou bien simplement disposé en un ou plusieurs points précis de cette circonférence. Dans un mode de réalisation particulier, l'élément de butée (10) destiné à bloquer le mouvement du clapet (4) soit vers le bas, soit vers le haut suivant le dispositif choisi, est une découpe particulière de la circonférence externe du premier clapet (4) de l'obturateur (11) et une découpe inverse de la circonférence interne de l'armature porteuse annulaire (2) dans sa partie haute. Les deux découpes étant destinées à coopérer de manière à bloquer de façon hermétique le soulèvement du clapet (4). On citera à titre d'exemple d'un tel système de butée grâce à des découpes particulières, le dispositif réalisé en un matériau semi-rigide tel le silicone décrit dans la demande de brevet européen EP 08872036. Le fonctionnement inverse à celui décrit dans EP 08872036 peut également être envisagé dans la présente invention, c'est-à-dïre que les clapets (4) et (5) vont coopérer pour se soulever à l'expiration du patient. Le clapet (5) va s'abaisser lors de l'inspiration. Dans le cas d'un tel système de découpe formant butée (10), la découpe en forme de biseau est préférée car elle permet d'assurer une meilleure étanchéité. Cependant toute autre forme permettant une telle coopération doit bien évidemment être considérée comme équivalente.

De telles découpes en biseau ou de toute autre forme équivalente sont parfaitement réalisables dans un matériau rigide tel le titane. Ce système de butée (10) présente l'avantage d'être simple à mettre en oeuvre, et il évite l'ajout d'éléments supplémentaires au dispositif (1) selon l'invention, et notamment à la partie active dudit dispositif.

Comme décrit dans la demande de brevet européen EP 08872036, les découpes décrites ci-dessus pour limiter le mouvement du clapet (4) par rapport à la partie supérieure de l'armature porteuse annulaire (2) peuvent également être utilisées pour rendre solidaires les clapets (4) et (5). Les découpes seront alors réalisées sur la circonférence intérieure du clapet (4) et sur la circonférence extérieure du clapet (5). Suivant le mode de réalisation choisi, à savoir l'abaissement conjoint des deux clapets (4) et (5), ou le soulèvement conjoint des deux clapets (4) et (5), les sens de découpe seront choisis « vers le haut » ou « vers le bas ».

Comme détaillé ci-dessus, les deux clapets (4) et (5) formant obturateur (11) ne sont actionnés que par les effets de dépression et de surpression découlant de la respiration du patient et, à contrario, doivent être immobiles suite à une pression autre comme le poids de tout élément étranger pouvant provenir, par exemple, du bol alimentaire. Pour répondre à cette contrainte, l'épaisseur de l'obturateur (11) est comprise entre 0,3 mm et 2,0 mm. Dans un mode de réalisation particulier, l'obturateur (11) a une épaisseur de 1,0 mm à 1,5 mm. Dans un mode de réalisation préféré, l'obturateur (11) a une épaisseur de 1,0 mm.

Par rapport aux valves et/ou clapets décrits dans l'art antérieur et généralement réalisés dans un matériau souple tel le silicone, les clapets (4) et (5) selon l'invention sont réalisés en titane massif ou dans un alliage à base de titane et sont fixés par l'intermédiaire d'une région charnière à une armature porteuse annulaire (2) elle aussi réalisée en titane massif ou dans un alliage à base de titane. Ces clapets sont donc particulièrement résistants lors de l'arrivée de fluides ou du bol alimentaire sur le dispositif, tout en entrant en mouvement suivant la respiration du patient.

Le poids du bol alimentaire est estimé en moyenne à 7,0 g ce qui se traduit par une pression n'excédant pas, en moyenne, 3.10⁻³ MPa. Le bol alimentaire est constitué d'aliments partiellement ou totalement mâchés et imprégnés de salive. Suite à la déglutition, ce bol alimentaire va parcourir l'oesophage pour atteindre finalement l'estomac. Sa masse va être plus ou moins diffuse suite à la mastication et à la déglutition et suivant la nature et le poids des aliments qui le constituent, son trajet dans l'oesophage va être plus ou moins rapide. En conditions physiologiques, on peut donc raisonnablement considérer que la totalité dudit bol alimentaire ne viendra pas atteindre un point précis du dispositif selon l'invention au même instant. Le dispositif à clapets selon l'invention est conçu pour résister à une charge moyenne de 7,0 g. et ceci est amplement suffisant pour un bon fonctionnement du dispositif à clapets.

Par ailleurs, la surpression exercée lors de l'expiration du patient et la dépression exercée lors de l'inspiration, sont de l'ordre de 10⁻² MPa, c'est-à-dire que dans les deux cas elles sont largement supérieures à la pression moyenne exercée par le bol alimentaire, environ 3.10⁻³ MPa. Il est alors aisé de comprendre que les clapets (4) et (5) pourront se soulever ou s'abaisser sans difficulté lors de la respiration du patient.

Plus particulièrement, le dispositif selon l'invention est caractérisé en ce que le premier clapet (4) ou le second clapet (5), suivant le sens de fonctionnement du dispositif, et leurs charnières sont dimensionnés de sorte à pouvoir supporter une charge inférieure à 4,0 g. sans se soulever et/ou s'abaisser, selon le mode de réalisation de l'invention choisi.

Selon une variante, il peut être souhaité que les résistances des deux clapets (4) et (5) soient différentes afin de pouvoir s'adapter, par exemple, à des patients présentant des problèmes respiratoires entraînant des différences de pressions entre l'inspiration et l'expiration. Dans ce cas précis, les dimensions des deux clapets (4) et (5), ainsi que des deux régions charnières (3) et (3') correspondantes, peuvent être différentes.

D'une manière générale, il est préféré que le premier clapet (4) et le second clapet (5) ainsi que lesdites première (3) et seconde (3') régions charnières présentent la même épaisseur. Ceci évite d'avoir des zones faisant saillie qui pourraient donner lieu à une accumulation de fluides ou de résidus indésirables. Par ailleurs, cette dernière forme d'exécution présente de nombreux avantages comme, par exemple, la réduction des coûts de fabrication.

Dans un mode de réalisation préféré selon l'invention, afin d'augmenter la résistance à la pression exercée par les fluides tout en n'empêchant pas le fonctionnement des clapets (4) et (5), un dispositif d'assistance pouvant être mécanique, électrique ou électronique est utilisé. Ledit dispositif d'assistance (7, 8) est placé au niveau de l'armature porteuse annulaire (2) et/ou au niveau du premier clapet (4). La présente invention porte également sur un dispositif (1) à clapets pour prothèse intralaryngée dont l'armature porteuse annulaire (2) et/ou le premier clapet (4) sont munis d'un dispositif d'assistance (7, 8) pouvant être mécanique, électrique ou électronique.

Dans un mode de réalisation particulier, le dispositif d'assistance (7, 8) décrit ci-dessus est un dispositif aimanté. Par dispositif aimanté ou élément aimanté, on entend un ou plusieurs aimants permanents de type lanthanides qui soient biocompatibles ou rendus biocompatibles par différents traitements connus de l'homme de l'art, ou encore enfermés hermétiquement dans un logement adapté prévu à cet effet. Ledit dispositif aimanté peut être placé soit sur le premier clapet, soit sur la surface interne de l'armature porteuse du dispositif selon l'invention. En vis-à-vis du ou des aimants quand le premier clapet est en position fermée, on place un élément métallique. Par élément métallique, on entend un ou plusieurs éléments métalliques susceptibles d'être aimantés qui sont placés de façon à entrer en contact avec le ou les aimants quand le premier clapet est en position fermée. La nature du dispositif aimanté pourra être adaptée à chaque situation en faisant varier le nombre d'aimants et/ou leur position par exemple. Il peut également être envisagé d'utiliser deux aimants qui se font face.

Dans un mode de réalisation particulier, la présente invention porte sur un dispositif à clapets pour prothèse intralaryngée (1) comportant un dispositif d'assistance (7, 8) qui consiste en un élément métallique disposé au niveau de l'armature porteuse annulaire (2) venant en contact avec un élément aimanté disposé au niveau du premier clapet (4). Dans un mode de réalisation préféré, la présente invention porte sur un dispositif à clapets pour prothèse intralaryngée (1) comportant un dispositif d'assistance (7, 8) qui consiste en un élément métallique disposé au niveau du premier clapet (4) venant en contact avec un élément aimanté disposé au niveau de l'armature porteuse annulaire (2).

Comme décrit ci-dessus, un dispositif d'assistance mécanique peut être positionné sur chacun des clapets afin de faciliter leur coopération dans le fonctionnement souhaité. Selon ce mode de réalisation la présente invention décrit un dispositif dans lequel le premier clapet (4) et le second clapet (5) comprennent un dispositif d'assistance de manière à permettre le soulèvement uniquement du second clapet (5) et l'abaissement simultané du premier clapet (4) et du second clapet (5). Dans un autre mode de réalisation, les clapets (4, 5) du dispositif selon l'invention comprennent un dispositif d'assistance de manière à permettre l'abaissement uniquement du second clapet (5) et le soulèvement simultané du premier clapet (4) et du second clapet (5).

Le dispositif aimanté décrit ci-dessus a une fonction d'assistance mécanique du dispositif à clapets (1) selon l'invention. De manière plus générale, il sera facile pour l'homme du métier de remplacer ou de compléter ce dispositif aimanté par tout dispositif assurant une fonction équivalente ou susceptible d'améliorer cette fonction, tel qu'un autre dispositif d'assistance pouvant être mécanique, électrique ou électronique.

En outre, les régions charnières (3) et (3') et les clapets (4) et (5) peuvent ne pas présenter les mêmes épaisseurs, ces dernières pouvant être adaptées à une situation précise dictée par l'état même du patient.

Pour pouvoir assurer le mouvement du ou des clapets tout en maintenant une parfaite étanchéité, les charnières (3) et (3') doivent être réalisées dans un matériau semi-rigide ou encore rigide. La mise au point des zones charnières réalisées en un matériau semi-rigide ou rigide relève d'une démarche très particulière car il faut à la fois que le mouvement soit rendu possible avec une faible pression respiratoire mais également que les charnières permettent aux clapets de résister à la pression du bol alimentaire ou de tout fluide qui viendrait appuyer sur l'obturateur avec plus ou moins de force. Par ailleurs, comme énoncé ci-dessus, il faut que les charnières résistent à la colonisation et assurent un mouvement régulier et pérenne des clapets. De plus la zone charnière doit assurer une parfaite stabilité des axes de rotation par rapport à l'armature porteuse du dispositif afin de garantir l'étanchéité à la fermeture. La présente invention propose plusieurs types de charnières susceptibles de répondre à toutes ces contraintes mécaniques. Les principes de fonctionnement ne seront pas les mêmes si l'on choisit un matériau semi-rigide, super élastique ou rigide.

Dans le premier cas de figure, par matériau semi-rigide ou super élastique on entend dans la présente invention un matériau suffisamment souple pour pouvoir assurer une certaine élasticité tout en étant assez rigide pour pouvoir résister et rester en forme sous l'effet d'une faible pression. Un tel matériau semi-rigide peut être sélectionné parmi le plastique, la gomme, une résine ou encore le silicone. Un matériau super élastique comme le nitinol peut également être envisagé. Un matériau préféré consiste en le silicone 70 Shore A. La charnière consiste alors en une languette de silicone fixée sur la partie extérieure de l'armature porteuse annulaire (2) par l'intermédiaire de deux tétons et d'une plaque en titane massif (6). L'autre extrémité de la languette semi-rigide est fixée sur les clapets (4) et (5) par tout moyen adéquat. On citera à titre d'exemple la perforation d'une petite partie du clapet, créant des orifices dans lesquels on coulera le matériau semi-rigide. Ce mécanisme présente l'intérêt d'être parfaitement fonctionnel et biocompatible. Dans un mode de réalisation particulier, la présente invention décrit un dispositif à clapets (1) comportant une portion distale formant armature porteuse annulaire (2) et une portion centrale formant obturateur (11), l'obturateur (11) comprend i) une partie périphérique formant un premier clapet (4) solidaire de l'armature porteuse annulaire (2) au niveau d'une première région charnière (3), et ii) une partie centrale formant un second clapet (5) solidaire du premier clapet au niveau d'une seconde région charnière (3'), et un moyen formant collerette ou jupe (9) pour la fixation et le retrait de la prothèse intralaryngée au sein du larynx dysfonctionnel situé en dessous de l'armature porteuse annulaire (2), caractérisé en ce que l'armature porteuse annulaire (2) ainsi que les deux clapets (4) et (5) formant obturateur (11) sont en métal massif, préférentiellement en titane ou en alliage à base de titane et que les régions charnières (3, 3') sont réalisées en un ou plusieurs matériaux semi-rigides ou super élastiques.

Dans le second cas, la charnière sera réalisée dans un matériau rigide. Par matériau rigide on entend un mécanisme tel une charnière mécanique, constituée d'un axe rigide qui vient s'imbriquer dans une découpe réalisée dans les clapets (4) et (5). L'axe rigide peut être réalisé en rubis ou dans un métal tel le titane ou un alliage à base de titane ou tout autre métal rigide biocompatible. D'une manière plus générale tous les mécanismes utilisés en horlogerie et réalisés à partir de matériaux biocompatibles peuvent être utilisés dans la mise en oeuvre de la présente invention. Ce mécanisme présente l'intérêt d'être robuste car réalisé dans des matériaux rigides et biocompatibles qui ne s'altèrent pas ou peu. Dans un mode de réalisation préféré, la présente invention décrit un dispositif à clapets (1) comportant une portion distale formant armature porteuse annulaire (2) et une portion centrale formant obturateur (11), l'obturateur (11) comprend i) une partie périphérique formant un premier clapet (4) solidaire de l'armature porteuse annulaire (2) au niveau d'une première région charnière (3), et ii) une partie centrale formant un second clapet (5) solidaire du premier clapet au niveau d'une seconde région charnière (3'), et un moyen formant collerette ou jupe (9) pour la fixation et le retrait de la prothèse intralaryngée au sein du larynx dysfonctionnel situé en dessous de l'armature porteuse annulaire (2), caractérisé en ce que l'armature porteuse annulaire (2) ainsi que les deux clapets (4) et (5) formant obturateur (11) sont en métal massif, préférentiellement en titane ou en alliage à base de titane et que les régions charnières (3, 3') sont réalisées en un ou plusieurs matériaux rigides.

Le moyen formant collerette ou jupe (9) qui est située en dessous de l'armature porteuse annulaire (2) a pour rôle de permettre la mise en place et le maintien de la prothèse intralaryngée au sein d'un larynx dysfonctionnel. Cette jupe (9) est constituée de trois parties. La première partie (12) est située en haut du dispositif (1) et fixée à l'armature porteuse annulaire (2) par l'intermédiaire de trous permettant une liaison optimale avec le matériau constituant ladite jupe (9), la seconde partie (13) dont le diamètre est plus faible que la première partie (12) est située en dessous de celle-ci, enfin la troisième partie (14) dont le diamètre est supérieur à celui de la partie (13) est située en dessous de la seconde partie (13) et comporte une découpe en biseau (15) ou tronc de cône. Les variations de diamètre entre les trois parties permettent d'assurer un maintien en position axiale de la prothèse une fois implantée car ceci lui permet de s'adapter à la géométrie de l'anatomie du patient.

La jupe (9) peut être réalisée en tout matériau semi-rigide biocompatible. Elle peut être réalisée dans un matériau plein ou sous forme de ressort ou « stent ». Dans un mode de réalisation préféré, la jupe (9) est réalisée en silicone. En effet, le silicone étant déformable, il permet de faire pénétrer la jupe dans l'orifice du larynx, du cricoïde et des cordes vocales, et ainsi de positionner la troisième partie (14) au sommet de la trachée, le cricoïde et les cordes vocales se situant au niveau de la seconde partie (13) et la première partie (12) se trouvant au-dessus des deux précédentes. Le plan supérieur de la prothèse est alors situé approximativement à une distance comprise entre 10 mm et 15 mm au-dessus du plan des aryténoïdes. Le biseau (15) ou tronc de cône, facilite la pénétration de l'ensemble du dispositif (1) selon l'invention dans le larynx du patient. La jupe (9) réalisée en silicone est également munie d'ergots (16) destinés à bloquer la prothèse en translation et en rotation une fois que celle-ci est mise en place au sein du larynx du patient. Le diamètre intérieur de la jupe (9) permet le passage d'une canule de 6,0 mm de diamètre afin que d'éventuels examens médicaux puissent être réalisés. Enfin l'intérieur de la jupe (9) comporte une surépaisseur plane sur toute la hauteur du dispositif (1) selon l'invention afin d'augmenter la rigidité du dispositif (1) lors de l'implantation. La majorité des arêtes de la jupe (9) est arrondie afin d'être atraumatique d'une part, et de faciliter l'évacuation des mucosités des voies respiratoires inférieures vers la zone pharyngée d'autre part.

### EXEMPLES

Plusieurs tests ont été réalisés sur deux prothèses intralaryngées pour larynx dysfonctionnel selon l'invention pour s'assurer que leurs propriétés mécaniques répondaient aux besoins des patients. Ces tests consistent en un test de fatigue de l'articulation ou zone charnière (3, 3') du dispositif selon l'invention, en un test de contrôle de l'étanchéité des clapets aux liquides et en un test de collage de l'obturateur (11). Le modèle de dispositif à clapets selon l'invention testé ici a les caractéristiques suivantes :
- les régions charnières (3) et (3') sont situées du même côté du dispositif ;
- l'articulation ou région charnière (3, 3') est réalisée en silicone.

### 1. Test de fatigue de l'articulation

Les clapets (4) et (5) sont solidaires d'une articulation en silicone (3, 3'), fixée sur l'armature porteuse annulaire en titane (2) du dispositif à clapets formant prothèse intralaryngée (1). Cette articulation (3, 3') permet aux clapets (4) et (5), d'une part d'être au repos en position fermée, d'autre part de permettre au petit clapet (5) de s'ouvrir vers le bas, lors de l'inspiration, et de permettre au grand clapet (4) et au petit clapet (5) de s'ouvrir simultanément vers le haut lors de l'expiration.

Sur un patient au repos, le rythme de la respiration est en moyenne de 12 fois par minute, soit 720 fois par heure et 17 280 fois par jour ou 518 400 par mois. Si l'on envisage une durée d'implantation d'au moins 3 mois chez les patients, avec un coefficient de sécurité de 2, cela représente 3 110 400 cycles de respiration donc de sollicitation de l'articulation (3, 3') en silicone.

Afin de s'assurer du coefficient de sécurité, les deux dispositifs selon l'invention testés ont été soumis à un test pneumatique d'ouverture et de fermeture des clapets (4) et (5) à la fréquence de 2 Hz et quelquefois de 1 Hz, pendant une durée supérieure à 4 millions de cycles. Le tableau 1 ci-dessous montre les résultats de l'essai pour un des dispositifs selon l'invention, avec des observations détaillées sous binoculaire à chaque arrêt.

**Tableau 1 : test de fatigue de l'articulation**

| **Etape** | **Départ phase (Date et Heure)** | **Fin phase (Date et Heure)** | **Durée phase (h:min:s)** | **Fréquence (Hz)** | **Nombre de cycles effectués** |
|---|---|---|---|---|---|
| 1 | 15/12/09 19:06 | 14:52 | 19:46:00 | 2 | 142 320 |
| 2 | 16/12/09 15:28 | 17/12/09 18:08 | 02:40:00 | 2 | 334 320 |
| 3 | 17/12/09 19:01 | 18:31 | 23:30:00 | 2 | 503 520 |
| 4 | 18/12/09 20:39 | 21/12/09 18:53 | 22:14:00 | 2 | 1 009 200 |
| 5 | 22/12/09 19:02 | 28/12/09 15:43 | 20:41:00 | 1 | 1 515 660 |
| 6 | 30/12/09 19:44 | 4/1/10 9:48 | 14:04:00 | 1 | 1 911 900 |
| 7 | 4/1/10 10:18 | 5/1/10 9:40 | 2322:00 | 2 | 2 080 140 |
| 8 | 5/1/10 11:06 | 8/1/10 15:05 | 03:59:00 | 2 | 2 627 220 |
| 9 | 8/1/10 16:22 | 11/1/10 15:46 | 23:24:00 | 2 | 3 141 300 |
| 10 | 11/1/10 17:06 | 13/1/10 14:20 | 21:14:00 | 2 | 3 466 980 |
| 11 | 13/1/10 14:20 | 14/1/10 8:33 | 18:13:00 | 1 | 3 532 560 |
| 12 | 15/1/10 12:22 | 18/1/10 14:45 | 02:23:00 | 2 | 4 068 120 |
| 13 | 18/1/10 16:44 | 17:47 | 01:03:00 | 2 | 4 075 680 |
| 14 | 19/1/10 9:35 | 19/1/10 10:20 | 00:45:00 | 2 | 4 081 080 |

On constate qu'au bout de plus de 4 millions de cycles, les deux dispositifs selon l'invention sont intacts, sans aucune amorce de fissure sur l'articulation en silicone (3, 3'). Les parties réalisées en titane ne présentent aucune altération. Les deux dispositifs sont par ailleurs parfaitement fonctionnels.

Les seules observations faites sont qu'il apparaît un léger matage dans les zones de contact entre les clapets et l'armature porteuse annulaire. Ceci est cependant sans conséquence sur le fonctionnement du dispositif selon l'invention.

### 2. Test d'étanchéité des clapets aux liquides

Comme cela est décrit dans la description, le dispositif selon l'invention est placé dans le larynx. Il est donc soumis au passage du bol alimentaire, et notamment aux liquides qui peuvent être de l'eau ou des liquides de différentes natures.

Ce test est réalisé à l'aide d'un papier buvard. Ce dernier est préalablement pesé puis placé sous les clapets (4) et (5). Une certaine quantité d'eau est ensuite versée sur les clapets (4) et (5) maintenus en position fermée donc formant obturateur (11). Les résultats sont détaillés ci-dessous dans le tableau 2.

Afin de définir un critère d'acceptation compatible avec les possibilités d'acceptation de la trachée en conditions physiologiques, il a été décidé que l'accroissement de masse du papier buvard devait rester inférieure à 1 % de la masse d'eau versée, correspondant au contenu de 5 cuillères à soupe, versées successivement, soit environ 62 grammes d'eau.

Ce test a été effectué à chaque arrêt pour observation de l'état de l'articulation ou zone charnière (3, 3') et de celui des clapets (4) et (5), au cours des essais de fatigue.

**Tableau 2 : test d'étanchéité aux liquides**

| | **Longueur Imbibée mesurée (cm)** | | **Masse d'eau correspondante (g)** | | | |
|---|---|---|---|---|---|---|
| **Nombre de cycles effectués** | **L1** | **L2** | **M1** | **M2** | **% de fuites 1** | **% de fuites 2** |
| 0 | - | - | - | - | - | - |
| 503 520 | - | - | - | - | - | - |
| 1 009 200 | - | - | - | - | - | - |
| 1 515 660 | 5 | 4 | 0,10 | 0,08 | 0,16 | 0,13 |
| 2 080 140 | 4,5 | 6,5 | 0,09 | 0,13 | 0,15 | 0,21 |
| 2 627 220 | 7 | 7 | 0,14 | 0,14 | 0,23 | 0,23 |
| 3 141 300 | 6 | 6 | 0,12 | 0,12 | 0,20 | 0,20 |
| 3 532 560 | 1 | 8 | 0,14 | 0,16 | 0,73 | 0,26 |
| 4 018 080 | 8,5 | 7,3 | 0,17 | 0,15 | 0,28 | 0,25 |

Les premières fuites sont mesurées à partir de 1 500 000 cycles de respiration. Les pourcentages de fuite sont très légers mais augmentent avec le nombre de cycles effectués.

Les fuites mesurées se situent toujours à des niveaux très bas, inférieurs à 0,28 % pour l'un des dispositifs et à 0,26 % pour l'autre dispositif. Ceci démontre la bonne étanchéité des deux clapets (4) et (5) formant obturateur (11) sur chacun des dispositifs testés.

### 3. Test de collage

Ces tests sont destinés à évaluer l'effet de certains produits visqueux ingérés par le patient, sur la force nécessaire à l'ouverture des deux clapets (4) et (5) lors du cycle respiratoire. Ces tests ont été menés en utilisant un mélange de 50% miel et de 50% d'eau assimilable à la viscosité d'un produit composé de miel et de salive lors de la phase de déglutition. Les mesures ont été effectuées à l'aide d'un dynamomètre lors de chaque arrêt des essais de fatigue pour contrôle. Les figures 7 et 8, relatives au petit clapet (5) et au grand clapet (4), montrent que la force d'ouverture ne varie pas notablement, au cours de la vie de l'implant.

La Fig. 7 montre que la force d'ouverture du petit clapet (5) ne varie pas notablement, au cours de la vie du dispositif selon l'invention.

La Fig. 8 montre que la force d'ouverture du grand clapet (4) ne varie pas notablement, au cours de la vie du dispositif selon l'invention.

### 4. Test de toux

Ceux-ci sont destinés à vérifier que les dispositifs selon l'invention, sous l'effet d'un réflexe de toux, s'ouvrent bien pour permettre à l'air expulsé de sortir et qu'ils retrouvent leur position de repos, en formant l'obturateur (11), sans déformation afin d'empêcher le bol alimentaire de pénétrer dans la trachée. Ce test a été réalisé en effectuant 100 impulsions correspondant à une toux lors de chacun des arrêts pour contrôle des dispositifs lors des essais de fatigue. Les résultats ont montré que les implants gardaient leur fonctionnalité et leur intégrité au terme de ces essais.

De manière générale, la présente description a pour but d'illustrer l'invention de la manière la plus claire possible et toute modification évidente de réalisation doit être considérée comme équivalente et, de ce fait, être considérée comme couverte par les revendications ci-après qui définissent l'étendue de la protection souhaitée.

## Revendications

1. Dispositif à clapets (1) destiné à être implanté au sein d'un larynx dysfonctionnel , ledit dispositif comportant une portion distale formant armature porteuse annulaire (2) et une portion centrale formant obturateur (11) destiné à permettre le passage de l'air et à empêcher de manière hermétique le passage de tout autre élément, l'obturateur (11) comprend i) une partie périphérique formant un premier clapet (4) solidaire de l'armature porteuse annulaire (2) au niveau d'une première région charnière (3), et ii) une partie centrale formant un second clapet (5) solidaire du premier clapet au niveau d'une seconde région charnière (3'), et un moyen formant collerette ou jupe (9) pour la fixation et le retrait de la prothèse intralaryngée au sein d'un larynx dysfonctionnel situé en-dessous de l'armature porteuse annulaire (2), **caractérisé en ce que** l'armature porteuse annulaire (2) ainsi que les deux clapets (4) et (5) formant obturateur (11) sont en métal massif.

2. Dispositif selon la revendication 1, **caractérisé en ce que** l'armature porteuse annulaire (2) ainsi que les deux clapets (4) et (5) formant obturateur (11) sont constitués de titane ou d'un alliage à base de titane.

3. Dispositif selon l'une quelconque des revendications 1 ou 2, **caractérisé en ce que** les régions charnières (3) et (3') sont situées entre les clapets (4) et (5) et l'armature porteuse annulaire (2) pour ne former qu'une seule zone charnière (3, 3') pour les deux clapets (4) et (5) .

4. Dispositif selon l'une quelconque des revendications 1 à 3, **caractérisé en ce que** les régions charnières (3) et (3') sont constituées d'un matériau semi-rigide, super élastique ou rigide.

5. Dispositif selon l'une quelconque des revendications 3 ou 4, **caractérisé en ce que** les régions charnières (3) et (3') sont constituées d'une languette en silicone bloquée par une plaque de titane (6) et que des orifices pratiqués dans la surface des clapets (4) et (5) permettent le passage du silicone pour un bon ancrage de la languette.

6. Dispositif selon l'une quelconque des revendications 3 ou 4, **caractérisé en ce que** les régions charnières (3) et (3') sont constituées d'un axe rigide sur lequel sont articulés les deux clapets (4) et (5) .

7. Dispositif selon la revendication 6, **caractérisé en ce que** les régions charnières (3) et (3') sont constituées d'un axe sur lequel sont articulés les deux clapets (4) et (5), ledit axe étant constitué d'un matériau sélectionné parmi le rubis, un métal tel le titane massif ou un alliage à base de titane, ou tout autre métal biocompatible.

8. Dispositif selon l'une quelconque des revendications 1 à 7, **caractérisé en ce que** la partie interne du clapet (4) comprend au moins un élément de butée (10) empêchant le soulèvement du clapet (5).

9. Dispositif selon l'une quelconque des revendications 1 à 8, **caractérisé en ce que** la partie interne de l'armature porteuse annulaire (2) comprend au moins un élément de butée (10) empêchant l'abaissement du clapet (4).

10. Dispositif selon l'une quelconque des revendications 8 ou 9, **caractérisé en ce que** l'élément de butée (10) consiste **en ce que** la circonférence externe du premier clapet (4) de l'obturateur (11) soit découpée en forme de biseau « vers le bas » et **en ce que** la circonférence interne de l'armature porteuse annulaire (2) se trouvant en vis-à-vis soit également découpée en biseau « inversé vers le haut », les deux découpes en biseau coopérant entre elles de manière à bloquer le soulèvement du premier clapet (4).

11. Dispositif selon l'une quelconque des revendications 1 à 10, **caractérisé en ce que** le premier clapet (4) et le second clapet (5) comprennent un dispositif d'assistance de manière à permettre le soulèvement uniquement du second clapet (5) et l'abaissement simultané du premier clapet (4) et du second clapet (5).

12. Dispositif selon l'une quelconque des revendications 1 à 11, **caractérisé en ce que** le premier clapet (4) et le second clapet (5) comprennent un dispositif d'assistance de manière à permettre l'abaissement uniquement du second clapet (5) et le soulèvement simultané du premier clapet (4) et du second clapet (5).

13. Dispositif selon la revendication 11 ou 12, **caractérisé en ce que** ledit dispositif d'assistance est un dispositif aimanté.

14. Dispositif selon l'une quelconque des revendications 1 à 13, **caractérisé en ce que** le moyen formant collerette ou jupe (9) pour la fixation et le retrait de la prothèse intralaryngée au sein d'un larynx dysfonctionnel situé en-dessous de l'armature porteuse annulaire (2) est en silicone.

15. Dispositif selon la revendication 14 **caractérisé en ce que** le moyen formant jupe (9) est constitué d'une première partie (12) située en haut du dispositif (1) et fixée à l'armature porteuse annulaire (2) par l'intermédiaire de trous permettant une liaison optimale avec le silicone , d'une seconde partie (13) dont le diamètre est plus faible que la première partie (12) et située en-dessous de celle-ci, et enfin d'une troisième partie (14) dont le diamètre est supérieur à celui de la partie (13) et située en-dessous de la seconde partie (13) et qui comporte une découpe en biseau (15), la surface extérieure de la jupe (9) étant munie d'ergots (16) et la surface intérieure de la jupe (9) comportant une surépaisseur plane sur toute sa hauteur.

## Patentansprüche

1. Ventilvorrichtung (1), die dazu bestimmt ist, in einen dysfunktionellen Larynx implantiert zu werden, wobei die Vorrichtung einen distalen Abschnitt, der eine ringförmige Trägerstruktur (2) bildet, und einen mittleren Abschnitt, der einen Verschluss (11) bildet, der dazu bestimmt ist, den Durchgang der Luft zu ermöglichen und hermetisch den Durchgang jedes anderen Elements zu verhindern, wobei der Verschluss (11) i) einen peripheren Teil, der ein erstes Ventil (4) bildet, das mit der ringförmigen Trägerstruktur (2) an einem ersten Scharnierbereich (3) fest verbunden ist, und ii) einen mittleren Teil aufweist, der ein zweites Ventil (5) bildet, das mit dem ersten Ventil an einem zweiten Scharnierbereich (3') fest verbunden ist, und ein Mittel aufweist, das einen Kragen oder eine Schürze (9) zum Befestigen und zum Entfernen der intralaryngealen Endoprothese in einem dysfunktionellen Larynx bildet, das unter der ringförmigen Trägerstruktur (2) angeordnet ist, **dadurch gekennzeichnet, dass** die ringförmige Trägerstruktur (2) und die zwei Ventile (4) und (5), die einen Verschluss (11) bilden, aus massivem Metall sind.

2. Vorrichtung nach Anspruch 1, **dadurch gekennzeichnet, dass** die ringförmige Trägerstruktur (2) und die zwei Ventile (4) und (5), die einen Verschluss (11) bilden, aus Titan oder einer Legierung auf Titan-Basis gebildet sind.

3. Vorrichtung nach einem der Ansprüche 1 oder 2, **dadurch gekennzeichnet, dass** die Scharnierbereiche (3) und (3') zwischen den Ventilen (4) und (5) und der ringförmigen Trägerstruktur (2) angeordnet sind, um nur einen einzigen Scharnierbereich (3, 3') für die zwei Ventile (4) und (5) zu bilden.

4. Vorrichtung nach einem der Ansprüche 1 bis 3, **dadurch gekennzeichnet, dass** die Scharnierbereiche (3) und (3') aus einem halbstarren, einem superelastischen oder starren Material gebildet sind.

5. Vorrichtung nach einem der Ansprüche 3 oder 4, **dadurch gekennzeichnet, dass** die Scharnierbereiche (3) und (3') aus einer Lasche aus Silikon gebildet sind, die durch eine Titanplatte (6) blockiert ist, und dass Öffnungen, die in der Oberfläche der Ventile (4) und (5) ausgeformt sind, den Durchgang von Silikon für eine gute Verankerung der Lasche ermöglichen.

6. Vorrichtung nach einem der Ansprüche 3 oder 4, **dadurch gekennzeichnet, dass** die Scharnierbereiche (3) und (3') aus einer starren Achse gebildet sind, mit der die zwei Ventile (4) und (5) gelenkig verbunden sind.

7. Vorrichtung nach Anspruch 6, **dadurch gekennzeichnet, dass** die Scharnierbereiche (3) und (3') aus einer Achse gebildet sind, mit der die zwei Ventile (4) und (5) gelenkig verbunden sind, wobei die Achse aus einem Material gebildet ist, das aus Rubin, einem Metall wie reinem Titan oder einer Legierung auf Titan-Basis oder aus einem beliebigen anderen biokompatiblen Metall ausgewählt ist.

8. Vorrichtung nach einem der Ansprüche 1 bis 7, **dadurch gekennzeichnet, dass** der innere Teil des Ventils (4) mindestens ein Anschlagelement (10) aufweist, das das Anheben des Ventils (5) verhindert.

9. Vorrichtung nach einem der Ansprüche 1 bis 8, **dadurch gekennzeichnet, dass** der innere Teil der ringförmigen Trägerstruktur (2) mindestens ein Anschlagelement (10) aufweist, das das Absenken des Ventils (4) verhindert.

10. Vorrichtung nach einem der Ansprüche 8 oder 9, **dadurch gekennzeichnet, dass** das Anschlagelement (10) darin besteht, dass der Außenumfang des ersten Ventils (4) des Verschlusses (11) in Form einer Abschrägung "nach unten" ausgeschnitten ist und dass der Innenumfang der ringförmigen Trägerstruktur (2), die gegenüberliegt, ebenfalls als "nach oben umgekehrte" Abschrägung ausgeschnitten ist, wobei die beiden Ausschnitte in Form von Abschrägungen miteinander derart zusammenwirken, um das Anheben des ersten Ventils (4) zu blockieren.

11. Vorrichtung nach einem der Ansprüche 1 bis 10, **dadurch gekennzeichnet, dass** das erste Ventil (4) und das zweite Ventil (5) eine Hilfsvorrichtung derart aufweisen, um das Anheben ausschließlich des zweiten Ventils (5) und das gleichzeitige Absenken des ersten Ventils (4) und des zweiten Ventils (5) zu ermöglichen.

12. Vorrichtung nach einem der Ansprüche 1 bis 11, **dadurch gekennzeichnet, dass** das erste Ventil (4) und das zweite Ventil (5) eine Hilfsvorrichtung derart aufweisen, um das Absenken ausschließlich des zweiten Ventils (5) und das gleichzeitige Anheben des ersten Ventils (4) und des zweiten Ventils (5) zu ermöglichen.

13. Vorrichtung nach Anspruch 11 oder 12, **dadurch gekennzeichnet, dass** die Hilfsvorrichtung eine magnetische Vorrichtung ist.

14. Vorrichtung nach einem der Ansprüche 1 bis 13, **dadurch gekennzeichnet, dass** das Mittel, das einen Kragen oder eine Schürze (9) zum Befestigen und zum Entfernen der intralaryngealen Endoprothese in einem dysfunktionellen Larynx bildet, das unter der ringförmigen Trägerstruktur (2) angeordnet ist, aus Silikon ist.

15. Vorrichtung nach Anspruch 14, **dadurch gekennzeichnet, dass** das Mittel, das eine Schürze (9) bildet, aus einem ersten Teil (12), der oben auf der Vorrichtung (1) angeordnet ist und an der ringförmigen Trägerstruktur (2) durch Löcher befestigt ist, die eine optimale Verbindung mit dem Silikon ermöglichen, einem zweiten Teil (13), dessen Durchmesser geringer als der erste Teil (12) ist und der unter diesem angeordnet ist, und schließlich aus einem dritten Teil (14) gebildet ist, dessen Durchmesser größer als jener des Teils (13) ist und der unter dem zweiten Teil (13) angeordnet ist und der einen Ausschnitt in Form von einer Abschrägung (15) aufweist, wobei die Außenfläche der Schürze (9) mit Spornen (16) ausgestattet ist und die Innenfläche der Schürze (9) eine flache Überdicke auf ihrer gesamten Höhe aufweist.

## Claims

1. A flap valve device (1) intended to be implanted in a dysfunctional larynx, said device comprising a distal portion forming an annular bearing structure (2) and a central portion forming a shutter (11) intended to allow air to pass and to hermetically prevent any other element from passing, the shutter (11) comprises i) a peripheral part forming a first valve flap (4) secured to the annular bearing structure (2) at a first hinge region (3), and ii) a central part forming a second valve flap (5) secured to the first valve flap at a second hinge region (3'), and a means forming a collar or skirt (9) for fixing the intralaryngeal prosthesis into, and removing it from, a dysfunctional larynx situated below the annular bearing structure (2), **characterized in that** the annular bearing structure (2) and the two valve flaps (4) and (5) that form the shutter (11) are made of solid metal.

2. The device as claimed in claim 1, **characterized in that** the annular bearing structure (2) and the two valve flaps (4) and (5) that form the shutter (11) are made of titanium or of a titanium-based alloy.

3. The device as claimed in either one of claims 1 and 2, **characterized in that** the hinge regions (3) and (3') are situated between the valve flaps (4) and (5) and the annular bearing structure (2) to form just one single hinge zone (3, 3') for the two valve flaps (4) and (5).

4. The device as claimed in any one of claims 1 to 3, **characterized in that** the hinge regions (3) and (3') are made of a semi-rigid, superelastic or rigid material.

5. The device as claimed in either one of claims 3 and 4, **characterized in that** the hinge regions (3) and (3') consist of a silicone tab blocked by a titanium plate (6) and that orifices made in the surface of the valve flaps (4) and (5) allow the passage of the silicone to ensure firm anchorage of the tab.

6. The device as claimed in either one of claims 3 and 4, **characterized in that** the hinge regions (3) and (3') consist of a rigid pin on which the two valve flaps (4) and (5) are articulated.

7. The device as claimed in claim 6, **characterized in that** the hinge regions (3) and (3') consist of a pin on which the two valve flaps (4) and (5) are articulated, said pin being made of a material selected from ruby, a metal such as solid titanium or a titanium-based alloy, or any other biocompatible metal.

8. The device as claimed in any one of claims 1 to 7, **characterized in that** the internal part of the valve flap (4) comprises at least one limit stop element (10) preventing the valve flap (5) from rising.

9. The device as claimed in any one of claims 1 to 8, **characterized in that** the internal part of the annular bearing structure (2) comprises at least one limit stop element (10) preventing the valve flap (4) from falling.

10. The device as claimed in either one of claims 8 and 9, **characterized in that** the limit stop element (10) consists of the fact that the external circumference of the first valve flap (4) of the shutter (11) is cut with a "downward" bevel, and that the internal circumference of the annular bearing structure (2) facing it is also cut with an "inverse upward" bevel, the two bevel cuts collaborating with one another to block the rise of the first valve flap (4).

11. The device as claimed in any one of claims 1 to 10, **characterized in that** the first valve flap (4) and the second valve flap (5) comprise an assistance device so as to allow the rising of only the second valve flap (5) and the simultaneous falling of the first valve flap (4) and of the second valve flap (5).

12. The device as claimed in any one of claims 1 to 11, **characterized in that** the first valve flap (4) and the second valve flap (5) comprise an assistance device so as to allow the falling of only the second valve flap (5) and the simultaneous rising of the first valve flap (4) and of the second valve flap (5).

13. The device as claimed in claim 11 or 11, **characterized in that** said assistance device is a magnetized device.

14. The device as claimed in any one of claims 1 to 13, **characterized in that** the means that forms the collar or skirt (9) for fixing the intralaryngeal prosthesis into, and removing it from, a dysfunctional larynx situated below the annular bearing structure (2) is made of silicone.

15. The device as claimed in claim 14, **characterized in that** the means forming a skirt (9) is made up of a first part (12) situated at the top of the device (1) and fixed to the annular bearing structure (2) using holes that allow optimal bonding with the silicone, of a second part (13) of a smaller diameter than the first part (12) situated below the latter, and finally of a third part (14) of a diameter greater than that of the part (13) and situated below the second part (13) and which comprises a bevel cut (15), the exterior surface of the skirt (9) being provided with lugs (16) and the interior surface of the skirt (9) having a planar additional thickness over its entire height.
